# EUROPEAN PATENT SPECIFICATION

(11) **EP 1 175 232 B1**
(45) Date of publication and mention of the grant of the patent: **16.07.2003**
(21) Application number: 00902830.9
(22) Date of filing: 18.02.2000
(51) Int. Cl.: A61L 9/12

(54) **RECEPTACLE FOR THE DIFFUSION OF A FRAGRANT LIQUID**
BEHÄLTER ZUM ZERSTÄUBEN EINES DUFTSTOFFES IN FLÜSSIGKEIT
RECIPIENT POUR LA DIFFUSION D'UN LIQUIDE PARFUME

(30) Priority: 05.05.1999 IT CO990016 U; 26.10.1999 IT MI992236
(43) Date of publication of application: 30.01.2002
(73) Proprietor: Giovannone, Alberto, 20020 Busto Garolfo (IT)
(72) Inventor: Giovannone, Alberto, 20020 Busto Garolfo (IT)
(86) International application number: IB0000175
(87) International publication number: WO00067807

(56) References cited:
- EP-A- 0 134 360
- FR-A- 2 766 530
- US-A- 4 889 286
- US-A- 5 755 381

## Description

This invention concerns the field of receptacles for the diffusion of the perfume of liquid fragrant essences therein contained. In particular, it refers to those which include a porous top, applied to the neck, which after absorbing part of the liquid therein contained, diffuse the perfume into the surroundings through evaporation.

In fact, as is known, on the market there are deodorants for the car and the ambient composed of a receptacle containing perfumed liquid essence and a perfume-diffusing top applied to its neck, whether or not acting as a lid.

For all products on the market, perfume diffusion is originated up by moistening the diffuser top by means of a dipping cylinder or wick made of wood, cord or other material which draws the fragrant liquid contained in the receptacle by capillary action towards the said perfume-diffusing top which may be made of wood, felt, cork, cardboard, etc., having various esthetical shapes.

With this methods the wick constantly absorbs and feeds the diffusing material. This, once saturated, discharges the excess of essence into the ambient, dripping on to underlying surfaces and causing damage (sometimes irreparable) to dashboards, etc.

Moreover, since it is not possible to control the flow, the essence is consumed rapidly, so limiting the length of perfume emanation.

The inventor of this invention has devised a diffusing receptacle without these drawbacks which, quite simply, makes it possible to dose the quantity of liquid reaching the above-mentioned top in porous material by merely turning the receptacle upside-down, without having to insert either wicks or other types of dipping elements.

In fact, the subject of this invention is composed of a diffusing receptacle of the type described in the preamble to claim 1 enclosed, characterised by the contents of the part characterising this claim.

Hereunder, some examples of the receptacle according to this invention are given in detail and with reference to the enclosed drawings which represent:
- figure 1 represents a frontal view of the receptacle according to the invention in a vertical position with the top facing upwards;
- figure 2 represents a frontal view of the receptacle according to figure 1, with the fragrant liquid in contact with the top;
- figure 3 represents a frontal view of another example of the receptacle according to the invention with a ball inside situated on the bottom of the receptacle when in a vertical position with the top facing upwards;
- figure 4 represents a frontal view of the example given in figure 3 with the receptacle upside-down and the ball closing the neck of the receptacle;
- figure 5 represents a frontal view of another example with the receptacle in a vertical position with the top facing upwards; of the two balls therein contained, one pushes downwards against the upper part of a segment in the neck of the receptacle, whilst the other lies on the bottom;
- figure 6 represents a view of the example given in figure 5, with the receptacle upside-down and one of the two balls pushing against the opposite side of the segment, whilst the other remains between the segment and the top;

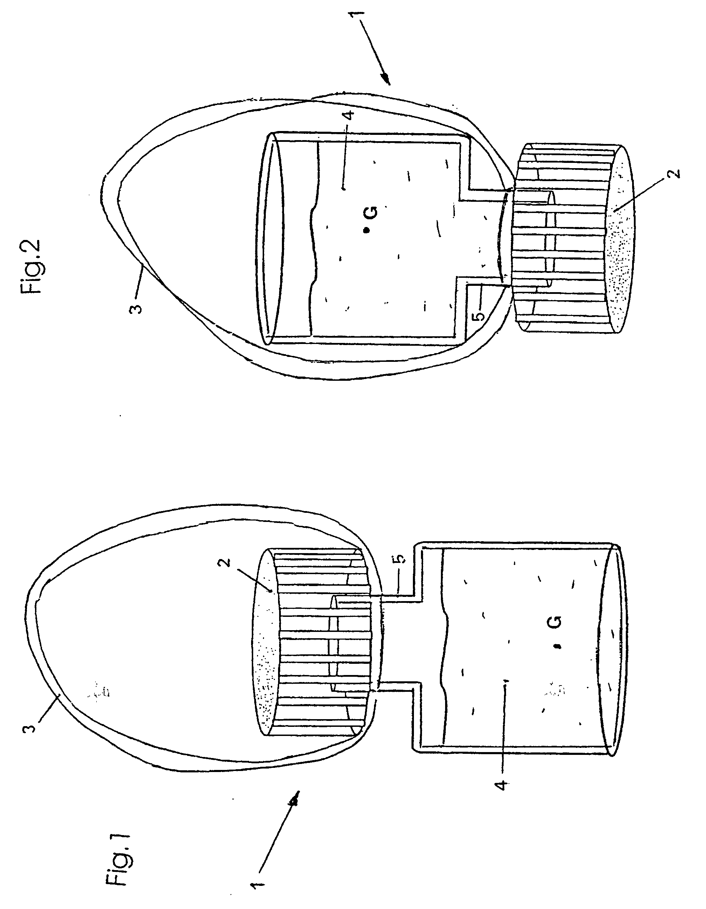

Considering first the figures 1 and 2, a first example of a diffusing receptacle 1 (hereafter called "receptacle") composed of a glass bottle with a neck 5 and a top 2 in porous material (preferable beech-wood or similar). It is to be noted that in this patent application, the word "neck" is used purely to indicate the open portion of the receptacle to which the top is fixed, without necessarily implying that this neck has a different diameter to the other parts of the receptacle, which may have varying forms and shapes.

The receptacle 1 is connected to some means of suspension, in this case a leather lace 3 or any other material used for this type of application. This lace 3 keeps the receptacle 1 in a vertical position, with the fragrant liquid 4 (hereafter called "liquid") therein contained not in contact with the porous top 2.

In this situation, the top 2, also acting as a lid, is dry and there is no emanation of perfume outside the receptacle 1.

If the receptacle is turned upside-down, as illustrated in figure 2, by turning it with respect to the suspension lace 3, the liquid 4 comes into contact with the top 2 which, being in a porous material, absorbs a certain amount and then slowly evaporates to the outside, diffusing the perfume of the essence contained in the liquid 4.

To reach this objective, it is sufficient to keep the receptacle 1 upside-down for a few seconds, since the porous material of which the top 2 is made, rapidly absorbs the liquid. After a few seconds, to avoid any superfluous liquid dripping out of the top, it is necessary to put the receptacle 1 back into its original position with the top 2 facing upwards. To facilitate this operation, the inventor has designed the receptacle 1 in such a way that its centre of gravity G and its top 2, in any position whatsoever and with any quantity whatsoever of liquid inside, are on opposite sides of the area where the suspension means are connected, in this case the lace 3, as can be seen in the enclosed drawings. This weight distribution ensures that, when the user leaves hold of the receptacle 1 a few seconds after having turned it upside-down, this receptacle, suspended by means of the lace 3 (obviously hanging from a hook or some other protruding element), revolves within the area where the lace 3 is connected, automatically returning to its original position with the top 2 facing upwards. So, the top 2 emanates the perfume for several days as a result of evaporation of the liquid it previously absorbed: when all the liquid has evaporated, it is sufficient to repeat the operations described above, and the cycle is repeated.

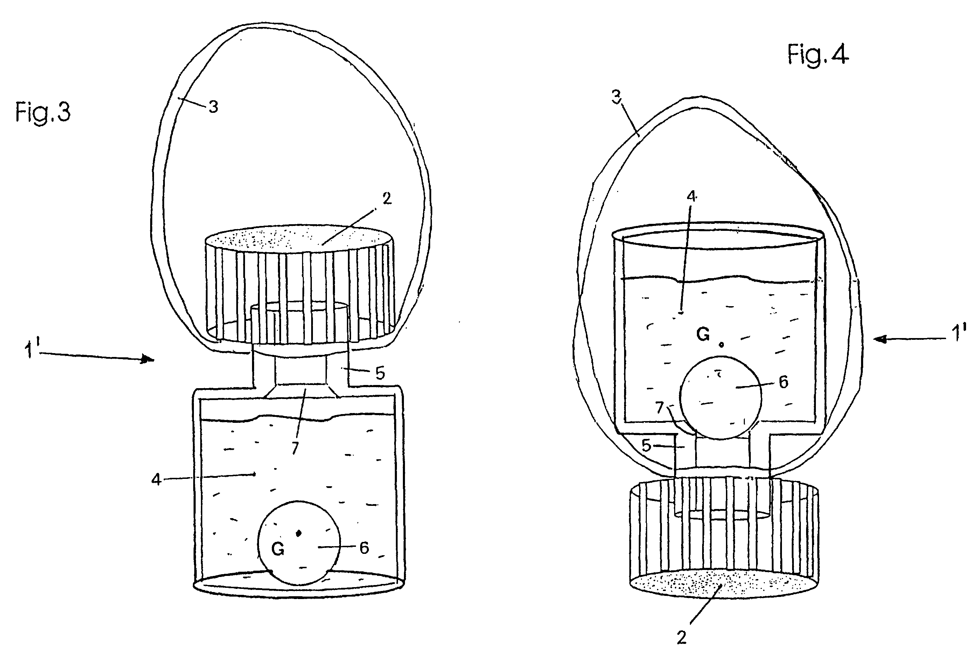

In order to avoid undesired dripping of the liquid from the top 2, resulting from the user keeping the receptacle 1 upside-down for too long, the inventor has provided for the insertion of a ball 6 into the receptacle 1' (see figures 3 and 4), made of a material with a higher specific gravity than that of the liquid 4, and the presence of an annular grooved area 7 in the neck 5 of the receptacle 1', of a size such that, when upside-down (fig 4), this ball pushes against it as a result of gravity, so creating sufficient sealing against any further passage of the liquid 4. In this way, the volume of liquid entering into contact with the porous top 2 is limited to the amount present in the area of the receptacle 1' between the annular area 7 and the top 2. When the receptacle 1' is facing upwards, the ball 6 is inconsequential and lies on the bottom of the receptacle 1', as can be seen in figure 3.

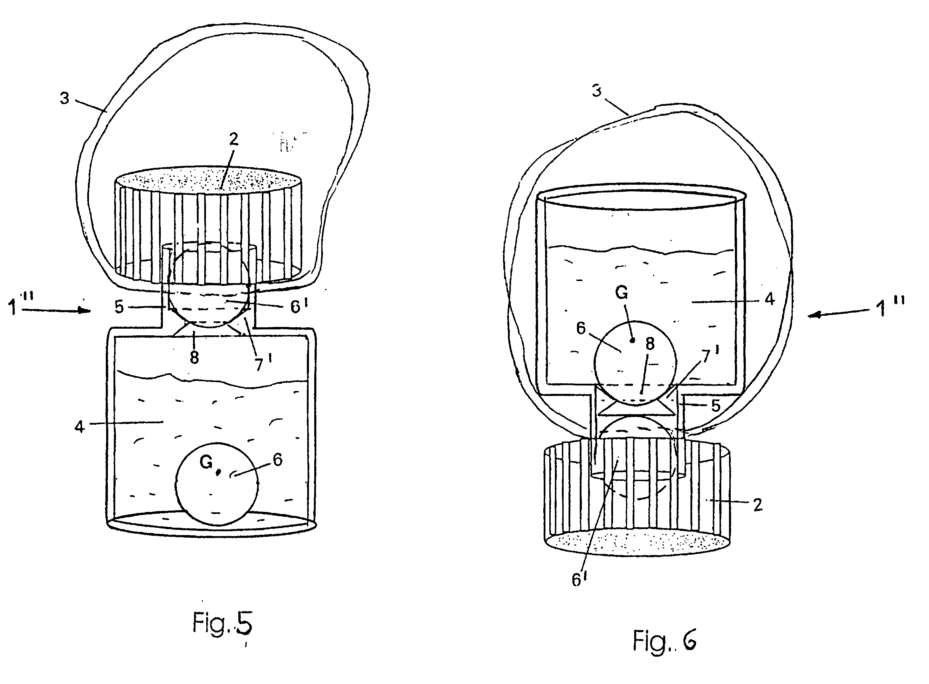

In a further design of a receptacle 1" according to the invention, the inventor has provided for the formation of the annular area of the neck 5 in the shape of a segment 7', as can be seen in figures 5 and 6, and the insertion of a second ball 6', similar to the one 6 described above, into that part of the receptacle 1" between the segment 7' and the top 2 in such a way that, when the receptacle 1" is facing upwards, the second ball 6', through the effect of gravity, pushes against the segment 7' which is directly opposite, so creating a seal which prevents the liquid present in the area between the segment 7' and the top 2 from flowing back. In this way, a single dose of the liquid 4 is isolated within this part of the receptacle and in contact with the top 2, so that it is not even necessary to turn the receptacle 1" upside-down for a few seconds to bring about absorption of a determined quantity of the fragrant liquid 4. As can be seen in figure 6, the other ball 6 carries out the function described above, i.e. limiting, when the receptacle 1" is upside-down, the quantity of liquid between the segment 7' and the top 2 to the minimum necessary to form the said dose.

Naturally, the type, shape and proportions of the various elements making up a receptacle according to the invention, may be varied with respect to the examples illustrated herein but always referring to that set forth in the enclosed claim 1, not going beyond the limits, therefore, of the protection granted by this patent application. For example, the suspension means may be of a different type, with metallic elements pivoted or hinged together, etc.

## Claims

1. Diffusing receptacle (1, 1', 1") including a neck (5) and a top (2) made of porous material and destined to absorb part of the fragrant liquid (4) contained in the receptacle (1, 1', 1"), and to diffuse its perfume through evaporation towards the outside ambient, **characterised by** that it is provided with suspension means (3) connected to it, capable of keeping the receptacle in a vertical position with the top (2) facing upwards, the said receptacle being so designed that whatever its position, its centre of gravity (G) and the top (2) are on opposite sides of the area to which the receptacle (1,1',1") is connected by these suspension means (3).

2. Receptacle according to claim 1, where these suspension means are composed of a lace (3) made of flexible material connected to the neck (5) of the above-mentioned receptacle (1).

3. Receptacle according to one of the previous claims, containing a ball (6) made of a material with a specific gravity greater than that of the fragrant liquid (4), said neck (5) having an annular grooved area (7, 7') of such a size that, when the receptacle (1) is upside-down, the ball (6) pushes co-axially against it so as to create sufficient sealing to prevent the fragrant liquid (4) from passing through in the direction of the top (2).

4. Receptacle according to claim 3, where this annular area on the neck (5) is a segment (7') with a hole running through (8), and the receptacle (1) contains a second ball (6'), placed on the opposite side of the segment (7') to this ball (6), both balls being made of a material with a specific gravity greater than that of the fragrant liquid (4) and of such a size as to seal the hole (8) immediately opposite it, so preventing the flow of liquid when it pushes against it through the effect of gravity.

## Patentansprüche

1. Behälter (1, 1', 1") zur Duftverbreitung, der einen Hals (5) und einen Deckel (2) aus porösem Material zur Aufnahme eines Teils der duftenden Flüssigkeit (4), die in dem Behälter selbst (1, 1', 1") enthalten ist, miteinschließt und der den Duft durch Verdunstung an die Umgebung abgibt, **dadurch gekennzeichnet, daß** er Aufhängevorrichtungen (3) hat, die mit dem Behälter verbunden sind und dazu dienen, den Behälter in seiner Aufrechtstellung mit dem genannten Deckel (2) nach oben gerichtet su halten, wobei der genannte Behälter so entworfen ist, daß, in welcher Stellung er sich auch immer befindet, sein Schwerpunkt (G) und der genannte Deckel (2) sich hinsichtlich des Bereichs, in dem der Behälter (1, 1', 1") an die genannten Aufhängevorrichtungen (3) verbunden ist, auf entgegengesetzter Seite befinden.

2. Behälter nach Patentanspruch 1, bei dem die genannten Aufhängevorrichtungen aus einem Band (3) aus flexiblem Material bestehen, das am Hals (5) des o.g. Behälters (1) angebracht ist.

3. Behälter nach einer der vorgehenden Patentansprüche, der in seinem Inneren eine Kugel (6) hat, die aus einem Material besteht, das ein höheres spezifisches Gewicht hat als die genannte duftende Flüssigkeit (4), wobei der genannte Hals (5) eine Ringzone (7, 7') hat, die eine solche Form und ein solches Ausmaß hat, daß die genannte Kugel (6) bei umgekipptem Behälter (1) coaxial gegen die Ringzone drückt und dadurch eine genügende Abdichtung entsteht, um das Durchdringen der duftenten Flüssigkeit (4) in Richtung des Deckels (2) zu verhindern.

4. Behälter nach Patentanspruch 3, bei dem die genannte Ringzone in seinem Hals (5) durch eine Scheidewand (7') mit einer Öffnung (8) gebildet ist und wo sich im Behälter (1) eine zweite Kugel (6') befindet, die gegenüber der genannten Kugel (6) auf der gegenüberliegenden Seite der Scheidewand (7') liegt, wobei jede der Kugeln (6', 6) aus einem Material besteht, das ein höheres spezifisches Gewicht als die duftende Flüssigkeit (4) hat und ein solches Ausmaß hat, daß die der Kugel direkt gegenüberliegenden Seite der Öffnung (8) durch sie abgedichtet wird und somit, wenn sie durch ihre Schwerkraft gegen die Öffnung preßt, den Durchgang der Flüssigkeit verhindert.

## Revendications

1. Récipient (1, 1', 1") diffuseur comprenant un col (5) et un couvercle (2) réalisé en matière poreuse et destiné à absorber une partie d'un liquide odorant (4) contenu dans ledit récipient (1, 1', 1") et à en répandre le parfum à l'extérieur par évaporation, **caractérisé par le fait qu'**il est muni de moyens de suspension (3) qui y sont reliés et aptes à soutenir ledit récipient en le maintenant en position verticale avec ledit couvercle 2 tourné vers le haut, ledit récipient étant réalisé de telle façon que, quelle que soit sa position, son centre de gravité (G) et le couvercle (2) se trouvent à l'opposé de la partie dans laquelle le récipient (1, 1', 1") est relié aux moyens de suspension (3).

2. Récipient conforme à la revendication 1, dans lequel lesdits moyens de suspension sont constitués d'un cordon (3) de matière souple, relié au col dudit récipient (1).

3. Récipient conforme à l'une des revendications précédentes, contenant une bille (6) réalisée dans une matière d'un poids spécifique supérieur à celui du liquide odorant (4), le col (5) présentant une partie circulaire (7, 7') dont la forme et la dimension sont telles que, lorsque le récipient (1) est renversé, ladite bille (6) presse coaxialement contre ladite partie circulaire, créant une étanchéité suffisante à empêcher le liquide odorant (4) de passer à travers elle en direction du couvercle (2).

4. Récipient conforme à la revendication 3, où ladite partie circulaire du col (5) est une cloison (7') que traverse un trou (8), ledit récipient (1) contenant une deuxième bille (6') située de l'autre côté de la cloison (7') par rapport à ladite bille (6), chacune des billes (6', 6) étant composées d'une matière dont le poids spécifique est supérieur à celui du liquide odorant (4) et d'une dimension telle qu'elle assure l'étanchéité sur la face dudit trou (8) qui lui est directement opposée, empêchant ainsi le passage du liquide, quand il presse contre elle par effet de la force de gravité.
